# EUROPEAN PATENT APPLICATION

(11) **EP 4 147 779 A1**
(43) Date of publication of application: **15.03.2023**
(21) Application number: 22180311.7
(22) Date of filing: 22.06.2022
(51) Int. Cl.: B01L 3/00, A61B 10/00, C12M 1/28, G01N 1/02

(54) **APPARATUS AND METHOD FOR DIAGNOSTIC TESTING**

(30) Priority: 09.09.2021 US 202117470388
(71) Applicant: Olo Health Oy, 20100 Turku (FI)
(72) Inventor: Juntunen, Etvi, Turku (FI)
(74) Representative: Aaltonen, Janne Lari Antero

(57) **Abstract**

Disclosed is an apparatus (100) comprising a housing (102), a first cavity (202), a volumetric pool (216) having a limited internal volume and a squeezing mechanism (204). The first cavity (202) supports a first strip (106) having a first end portion (118) for receiving a fluid substrate and a second end portion (120). The squeezing mechanism is disposed corresponding to at least a portion of a second width (W) of the first cavity in a lateral direction (Z). When the first strip is drawn along a longitudinal direction (X) such that the first end portion moves into the first cavity, the first end portion is squeezed by the squeezing mechanism to squeeze out the fluid substrate to be transferred to the volumetric pool. The fluid substrate excess to the limited internal volume of the volumetric pool overflows from the volumetric pool to retain a desired volume.

## Description

### TECHNICAL FIELD

The present disclosure relates, generally, to testing kits, such as an immunochromatographic diagnostic test kit, and more specifically, to an apparatus and a method for diagnostic testing providing a desired volume of a fluid substrate for testing purposes.

### BACKGROUND

To a significant extent, many known tests presently available for detecting and measuring biomarkers are either time consuming, labour intensive, or in need of instrumental assistance to read results. Most known tests also lack an acceptable degree of sensitivity or specificity. This is unfortunate since rapid testing is important for diagnosis and treatment of various physiological conditions; detection of certain strains of micro-organisms; determination of the most appropriate antibiotic treatment of a patient; detection of drug analytes in individuals; detection of cancer cells in a patient's bio-fluid; detection of antibody to a microbial agent; detection of a disease-state protein; and the like. In recent years the in vitro diagnostics (IVD) industry has made enormous efforts to develop immunochromatographic tests.

Immunochromatographic diagnostic test kit usually includes an assay strip associated with one or more indicators and which may measure concentration and/or pH of a liquid and reveal the same by changing colour of the indicators according to the concentration and/or pH of the liquid. The aforesaid characteristic of the assay strips makes it useful for applications such as, urine infection test, blood test for determining sugar levels and the like. Such tests have found applications in both clinical and non-clinical fields. The rapid immunochromatographic test devices for diagnostic purposes are easy to operate and thus do not only contribute to the comfort of professional users, e.g., medical staff, but also allow the operation by non-professionals users, e.g., most patients.

Typically, a test sample of the liquid is applied on one end of the assay strip and the indicator is read to determine test results. For example, in order to assess hormonal balance such as, cortisol from saliva, a patient may be requested to lock or spit at one end of the assay strip and the indicators results may be read to quantify hormones. However, in some applications an exact amount of the test sample may need to be applied to one end of the assay strip. Too little test sample or too much test sample may affect the test results. Conventional assay strips do not provide techniques for applying the exact amount of the test sample and hence, the testing results may sometimes be incorrect. Solutions with external volume measurement functions exist which may be used to collect and transfer a defined amount of test sample to a corresponding immunochromatographic diagnostic test kit. However, use of external liquid transfer devices may add to the complexity of user experience and may thus hinder wide adoptions of such devices.

Therefore, in light of the foregoing discussion, there still exists a need to overcome the aforementioned drawbacks associated with known techniques for applying exact amount of the test sample to the immunochromatographic diagnostic test kit.

### SUMMARY

The aspects of the disclosed embodiments are directed to providing a solution that overcomes at least partially the problems encountered in the known techniques. The present disclosure provides an apparatus and a method for diagnostic testing which retains a desired volume of a fluid substrate to be used for testing purposes. Another aspect of the disclosed embodiments is to implement the present solutions in a portable immunochromatographic diagnostic test kit.

In one aspect, an embodiment of the present disclosure provides an apparatus for diagnostic testing, the apparatus comprising:
- a housing having a first length along a longitudinal direction thereof, a first height along a vertical direction thereof and a first width along a lateral direction thereof;
- a first cavity formed in the housing, the first cavity extending along the first length in the longitudinal direction and having a second width along the lateral direction, the first cavity adapted to support a first strip to be movable along the longitudinal direction therein, the first strip having a first end portion and a second end portion with the first end portion thereof located outside of the housing and adapted to receive a fluid substrate thereat when supported in the first cavity;
- a volumetric formed in the housing, the volumetric pool having a sidewall extending to a second height along the vertical direction to define a limited internal volume for the volumetric pool;
- a squeezing mechanism arranged in the housing, the squeezing mechanism disposed corresponding to at least a portion of the second width of the first cavity in the lateral direction; and
- wherein when the first strip is drawn along the longitudinal direction such that the first end portion moves into the first cavity, the first end portion is squeezed by the squeezing mechanism to squeeze out the fluid substrate received thereat to be transferred to the volumetric pool, and
- wherein the fluid substrate excess to the limited internal volume of the volumetric pool overflows from the volumetric pool, to retain a desired volume of the fluid substrate in the volumetric pool equivalent to the limited internal volume.

In another aspect, an embodiment of the present disclosure provides a method for diagnostic testing, the method comprising:
- providing a housing having a first cavity to support a first strip with a first end portion thereof located outside the housing and a second cavity to support a second strip with a third end portion thereof located inside the housing;
- receiving a fluid substrate at the first end portion of the first strip when supported in the first cavity; and
- drawing the first strip along the longitudinal direction such that the first end portion moves into the first cavity to be squeezed by a squeezing mechanism arranged in the housing, to squeeze out the fluid substrate received thereat to be transferred to a volumetric pool formed in the housing,
- wherein the volumetric pool has a limited internal volume to cause the fluid substrate excess to the limited internal volume of the volumetric pool to overflow from the volumetric pool, to retain a desired volume of the fluid substrate in the volumetric pool equivalent to the limited internal volume.

Embodiments of the present disclosure substantially eliminate or at least partially address the aforementioned problems in the prior art, and allow to retain only the desired volume of the fluid substrate in the volumetric pool equivalent to the limited internal volume by allowing overflow of the fluid substrate excess to the limited internal volume of the volumetric pool from the volumetric pool.

Additional aspects, advantages, features and objects of the present disclosure will be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF DRAWINGS

Embodiments of the present disclosure will now be described, by way of example only, with reference to the following diagrams wherein:
FIG. 1 is a perspective view illustration of an apparatus, in accordance with an embodiment of the present disclosure;
FIG. 2 is a perspective view illustration of the apparatus of FIG. 1 with a top section removed therefrom, in accordance with an embodiment of the present disclosure;
FIG. 3 is a top planar view illustration of the apparatus of FIG. 1 with the top section removed therefrom, in accordance with an embodiment of the present disclosure;
FIG. 4 is a top planar view illustration of the apparatus of FIG. 1, in accordance with an embodiment of the present disclosure;
FIG. 5 is a section view illustration of the apparatus sectioned along a plane A-A' of FIG. 4, in accordance with an embodiment of the present disclosure;
FIG. 6 is a section view illustration of the apparatus sectioned along a plane B-B' of FIG. 4, in accordance with an embodiment of the present disclosure;
FIGs. 7A-7E are section view illustrations of the apparatus depicting, in sequence, steps involved in a process of operating the apparatus, in accordance with an embodiment of the present disclosure;
FIG. 8A is a top planar view illustration of the apparatus with a first strip in a position depicting a first status indicator thereof, in accordance with an embodiment of the present disclosure;
FIG. 8B is a top planar view illustration of the apparatus with a first strip in a position depicting a second status indicator thereof, in accordance with an embodiment of the present disclosure;
FIG. 9 is a schematic illustration of an electrical circuit, in accordance with an embodiment of the present disclosure; and
FIG. 10 is a flowchart listing steps involved in a method for diagnostic testing, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In one aspect, an embodiment of the present disclosure provides an apparatus comprising:
- a housing having a first length along a longitudinal direction thereof, a first height along a vertical direction thereof and a first width along a lateral direction thereof;
- a first cavity formed in the housing, the first cavity extending along the first length in the longitudinal direction and having a second width along the lateral direction, the first cavity adapted to support a first strip to be movable along the longitudinal direction therein, the first strip having a first end portion and a second end portion with the first end portion thereof located outside of the housing and adapted to receive a fluid substrate thereat when supported in the first cavity;
- a volumetric formed in the housing, the volumetric pool having a sidewall extending to a second height along the vertical direction to define a limited internal volume for the volumetric pool;
- a squeezing mechanism arranged in the housing, the squeezing mechanism disposed corresponding to at least a portion of the second width of the first cavity in the lateral direction; and
- wherein when the first strip is drawn along the longitudinal direction such that the first end portion moves into the first cavity, the first end portion is squeezed by the squeezing mechanism to squeeze out the fluid substrate received thereat to be transferred to the volumetric pool, and
- wherein the fluid substrate excess to the limited internal volume of the volumetric pool overflows from the volumetric pool, to retain a desired volume of the fluid substrate in the volumetric pool equivalent to the limited internal volume.

In another aspect, an embodiment of the present disclosure provides a method of using an immunochromatographic diagnostic test kit, the method comprising:
- providing a housing having a first cavity to support a first strip with a first end portion thereof located outside the housing and a second cavity to support a second strip with a third end portion thereof located inside the housing;
- receiving a fluid substrate at the first end portion of the first strip when supported in the first cavity; and
- drawing the first strip along the longitudinal direction such that the first end portion moves into the first cavity to be squeezed by a squeezing mechanism arranged in the housing, to squeeze out the fluid substrate received thereat to be transferred to a volumetric pool formed in the housing,
- wherein the volumetric pool has a limited internal volume to cause the fluid substrate excess to the limited internal volume of the volumetric pool to overflow from the volumetric pool, to retain a desired volume of the fluid substrate in the volumetric pool equivalent to the limited internal volume.

The present disclosure relates to an apparatus for diagnostic testing. Herein, the apparatus may be a device that may collect and provide an exact amount of a fluid substrate, in which collected fluid substrate may be used for diagnostic testing purposes. It may be noted that a time when a desired volume of the fluid substrate may be needed for obtaining accurate testing result, the received fluid substrate may be in excess. For example, if the fluid substrate is saliva and a patient spit in excess saliva, the testing result may be compromised. In such cases, asking the patient to collect only the desired volume of the saliva may be a futile task. In order to get rid of the aforesaid problems, the apparatus of the present disclosure may be employed.

In the present examples, the apparatus is an immunochromatographic diagnostic test kit. It may be appreciated that the immunochromatographic diagnostic test kit may employ chromatography and immunoassay techniques for testing purposes. Herein, chromatography may be a technique for separating components such as, solutes from a solvent. The immunoassay technique may detect presence of and/or concentration of micromolecules such as, a protein in antigens or antibodies. The immunochromatographic diagnostic test kit may employ chromatography and immunoassay techniques for testing such as, but not limited to, pregnancy, parasitic infections such as, malaria and the like and bacterial infections such as, mycoplasma pneumoniae and the like, viral infections such as, influenza A/B, hepatitis B, hepatitis C infection and HIV, stress level and the like.

The apparatus comprises a housing having a first length along a longitudinal direction thereof, a first height along a vertical direction thereof and a first width along a lateral direction thereof. Herein, the housing may be in the form of a, generally, hollow cassette which houses internal components of the apparatus. The housing may have the first length along the longitudinal direction, the first height along the vertical direction and the first width along the lateral direction. In an embodiment, the first length, the first height and the first width of the housing may be 80 millimetres, 5 millimetres and 20 millimetres respectively.

The apparatus comprises a first cavity formed in the housing. The first cavity extends along the first length in the longitudinal direction and has a second width along the lateral direction. The first cavity is adapted to support a first strip to be movable along the longitudinal direction therein. The first strip has a first end portion and a second end portion with the first end portion thereof located outside of the housing and adapted to receive a fluid substrate thereat when supported in the first cavity. Herein, the first cavity may be in the form of a protrusion formed throughout along the first length in the longitudinal direction. The first cavity may have the second width along the lateral direction. The second width is lesser than the first width. The first cavity may support the first strip, i.e., the first strip may be disposed in the first cavity. Herein, the first strip may also be referred to as a sample collection strip or a wettable sample absorption pad, and may generally be composed of 30 percent cotton and 70 percent cellulose, for sample intake. The first strip may have the first end portion and the second end portion. The first end portion may be located outside the housing for receiving the fluid substrate such as, but not limited to, saliva, blood, urine, food/drink, water sample, brewing process sample and industrial process sample. For example, the first end portion of the first strip may be inserted in saliva collection tube or directly into a mouth for receiving the fluid substrate which, in this case, is the saliva, on the first end portion. The first strip may be movable along the longitudinal direction so as to bring the first end portion inside the first cavity of the housing, when required.

The apparatus comprises a volumetric pool formed in the housing. The volumetric pool has a sidewall extending to a second height along the vertical direction to define a limited internal volume for the volumetric pool. Herein, the volumetric pool may be in the form of a depression or a pit formed in the housing having the sidewall of the second height in vertical direction to define the limited internal volume. In an example, the volumetric pool may have a single continuous sidewall such as with a circular or similar cross-section, or may have multiple sidewalls joined together to define the volumetric pool. In the present apparatus, the second height of the sidewall of the volumetric pool is less than the first height of the housing such that the volumetric pool is located within the housing. The volumetric pool may collect desired volume of the first substrate received on the first end portion of the first strip which may be equivalent to the limited internal volume. For obtaining accurate testing results only the desired volume of the first substrate may need to be used for testing purposes. The first substrate excess to the limited internal volume may be discarded. Hence, the limited internal volume of the volumetric pool is defined based on (i.e., equivalent to) the desired volume of the fluid substrate. That is, the limited internal volume of the volumetric pool may be designed as larger or smaller, for example by varying the second height of the sidewall of the volumetric pool, depending on the desired volume of the fluid substrate.

Optionally, the defined limited internal volume of the volumetric pool is in a range of 10 µL to 200 µL. Herein, the range of the defined limited internal volume of the volumetric pool may be from 10 µL, 11 µL, 12 µL, 13 µL, 14 µL, 15 µL, 16 µL, 17 µL, 18 µL, 19 µL, 20 µL, 21 µL, 22 µL, 23 µL, 24 µL, 25 µL, 26 µL, 27 µL, 28 µL, 29 µL, 30 µL, 31 µL, 32 µL, 33 µL, 34 µL, 35 µL, 36 µL, 37 µL, 38 µL, 39 µL, 40 µL, 41 µL, 42 µL, 43 µL, 44 µL, 45 µL, 46 µL, 47 µL, 48 µL, 49 µL, 50 µL, 51 µL, 52 µL, 53 µL, 54 µL, 55 µL, 56 µL, 57 µL, 58 µL, 59 µL, 60 µL, 61 µL, 62 µL, 63 µL, 64 µL, 65 µL, 66 µL, 67 µL, 68 µL, 69 µL, 70 µL, 71 µL, 72 µL, 73 µL, 74 µL, 75 µL, 76 µL, 77 µL, 78 µL, 79 µL, 80 µL, 81 µL, 82 µL, 83 µL, 84 µL, 85 µL, 86 µL, 87 µL, 88 µL, 89 µL, 90 µL, 91 µL, 92 µL, 93 µL, 94 µL, 95 µL, 96 µL, 97 µL, 98 µL, 99 µL, 100 µL, 101 µL, 102 µL, 103 µL, 104 µL, 105 µL, 106 µL, 107 µL, 108 µL, 109 µL, 110 µL, 111 µL, 112 µL, 113 µL, 114 µL, 115 µL, 116 µL, 117 µL, 118 µL, 119 µL, 120 µL, 121 µL, 122 µL, 123 µL, 124 µL, 125 µL, 126 µL, 127 µL, 128 µL, 129 µL, 130 µL, 131 µL, 132 µL, 133 µL, 134 µL, 135 µL, 136 µL, 137 µL, 138 µL, 139 µL, 140 µL, 141 µL, 142 µL, 143 µL, 144 µL, 145 µL, 146 µL, 147 µL, 148 µL, 149 µL , 150 µL , 151 µL , 152 µL , 153 µL , 154 µL , 155 µL , 156 µL , 157 µL , 158 µL , 159 µL , 160 µL , 161 µL , 162 µL , 163 µL , 164 µL , 165 µL , 166 µL , 167 µL , 168 µL , 169 µL , 170 µL , 171 µL , 172 µL , 173 µL , 174 µL , 175 µL , 176 µL , 177 µL , 178 µL , 179 µL , 180 µL , 181 µL , 182 µL , 183 µL , 184 µL , 185 µL , 186 µL , 187 µL , 188 µL , 189 µL , 190 µL , 191 µL , 192 µL, 193 µL , 194 µL , 195 µL , 196 µL , 197 µL , 198 µL and 199 µL up to 11 µL, 12 µL, 13 µL, 14 µL, 15 µL, 16 µL, 17 µL, 18 µL, 19 µL, 20 µL, 21 µL, 22 µL, 23 µL, 24 µL, 25 µL, 26 µL, 27 µL, 28 µL, 29 µL, 30 µL, 31 µL, 32 µL, 33 µL, 34 µL, 35 µL, 36 µL, 37 µL, 38 µL, 39 µL, 40 µL, 41 µL, 42 µL, 43 µL, 44 µL, 45 µL, 46 µL, 47 µL, 48 µL, 49 µL, 50 µL, 51 µL, 52 µL, 53 µL, 54 µL, 55 µL, 56 µL, 57 µL, 58 µL, 59 µL, 60 µL, 61 µL, 62 µL, 63 µL, 64 µL, 65 µL, 66 µL, 67 µL, 68 µL, 69 µL, 70 µL, 71 µL, 72 µL, 73 µL, 74 µL, 75 µL, 76 µL, 77 µL, 78 µL, 79 µL, 80 µL, 81 µL, 82 µL, 83 µL, 84 µL, 85 µL, 86 µL, 87 µL, 88 µL, 89 µL, 90 µL, 91 µL, 92 µL, 93 µL, 94 µL, 95 µL, 96 µL, 97 µL, 98 µL, 99 µL, 100 µL, 101 µL, 102 µL, 103 µL, 104 µL, 105 µL, 106 µL, 107 µL, 108 µL, 109 µL, 110 µL, 111 µL, 112 µL, 113 µL, 114 µL, 115 µL, 116 µL, 117 µL, 118 µL, 119 µL, 120 µL, 121 µL, 122 µL, 123 µL, 124 µL, 125 µL, 126 µL, 127 µL, 128 µL, 129 µL, 130 µL, 131 µL, 132 µL, 133 µL, 134 µL, 135 µL, 136 µL, 137 µL, 138 µL, 139 µL, 140 µL, 141 µL, 142 µL, 143 µL, 144 µL, 145 µL, 146 µL, 147 µL, 148 µL, 149 µL , 150 µL , 151 µL , 152 µL , 153 µL, 154 µL , 155 µL , 156 µL , 157 µL , 158 µL , 159 µL , 160 µL , 161 µL , 162 µL , 163 µL, 164 µL , 165 µL , 166 µL , 167 µL , 168 µL , 169 µL , 170 µL , 171 µL , 172 µL , 173 µL, 174 µL , 175 µL , 176 µL , 177 µL , 178 µL , 179 µL , 180 µL , 181 µL , 182 µL , 183 µL, 184 µL , 185 µL , 186 µL , 187 µL , 188 µL , 189 µL , 190 µL , 191 µL , 192 µL , 193 µL, 194 µL , 195 µL , 196 µL , 197 µL , 198 µL, 199 µL and 200 µL. The said range of the defined limited internal volume of the volumetric pool may be sufficient for storing the desired volume of the fluid substrate received on the first end portion of the first strip for majority of the in-vitro diagnostic tests. In an embodiment, the limited internal volume is 60 µL with a deviation of about ± 5 percent. In an example, for serological blood test or HIV test, 2 µL of fingerpick blood diluted in larger volume may be the desired volume and the limited internal volume of the volumetric pool may accordingly be defined. In another example, for testing of hepatitis 80 µL of venous whole blood may be desired volume of the fluid sample, and thus the limited internal volume of the volumetric pool may be defined as 80 µL. The volumetric pool may also be used for drying chemistry components such as, buffers, salts, detergents, reporter particles, antibodies and antibody conjugates, as may be contemplated by a person skilled in the art.

The apparatus further comprises a squeezing mechanism arranged in the housing. The squeezing mechanism is disposed corresponding to at least a portion of the second width of the first cavity in the lateral direction. Herein, the squeezing mechanism may squeeze the first end portion of the first strip. It may be noted that the first strip may have varying thickness such that the first end portion of the first strip may be thicker as compared to the second end portion of the first strip. The varying thickness of the first strip may facilitate squeezing of only the first end portion of the first strip by the squeezing mechanism. In an embodiment, the squeezing mechanism may be in the form of two protrusions disposed opposite to each other, corresponding to at least the portion of the second width of the first cavity in the lateral direction such that, when the first end portion of the first strip passes over the squeezing mechanism, a constriction of space may be created that may ultimately squeeze the first end portion of the first strip. Since, the first end portion of the first strip is thicker, only the first end portion may face constriction when passing over the squeezing mechanism. In an alternative embodiment, the squeezing mechanism may include an active mechanism that may push linear actuators in order to create pressure on the first end portion of the first strip for squeezing the first end portion. It may be noted that when the first end portion with received fluid substrate is squeezed, the received fluid substrate may be extracted (squeezed) out from the first end portion of the first strip, and such extracted fluid substrate may then be transferred to and collected in the volumetric pool.

In one or more embodiments, the apparatus further comprises a transfer canal formed in the housing. The transfer canal disposes a region in the first cavity proximal to the squeezing mechanism in fluid communication with the volumetric pool. The transfer canal may be in the form of a channel formed in a region in the first cavity proximal to the squeezing mechanism, so as to collect the fluid substrate squeezed from the first end portion of the first strip thereby. The transfer canal may further be in fluid communication with the volumetric pool for transferring the collected fluid substrate obtained by squeezing the first end portion of the first strip thereto. For example, the transfer canal may lead to and be connected at a top, a middle or a lower portion of the sidewall of the volumetric pool. Since, the volumetric pool may be of limited internal volume, only limited internal volume of squeezed first substrate may be stored in the volumetric pool.

Optionally, the apparatus further comprises one or more overflow channels formed in the housing, the one or more overflow channels disposed in fluid communication with the sidewall of the volumetric pool at the second height thereof and with an excess pool formed in the housing, wherein the fluid substrate excess to the limited internal volume of the volumetric pool overflows from the volumetric pool to the excess pool via the one or more overflow channels. Herein, one or more overflow channels may be in the form of one or more conduits that may allow the fluid substrate excess to the limited internal volume to flow out (overflow) from the volumetric pool in order to keep the desired volume exactly right. Herein, the excess pool may be in the form of a pit formed in the housing so as to collect the fluid substrate excess to the limited internal volume. One end of the one or more overflow channels may be connected to the sidewall of the volumetric pool at the second height thereof and other end of the one or more overflow channels may be connected to the excess pool so as to transfer the fluid substrate excess to the limited internal volume from the volumetric pool to the excess pool.

It may be noted that when the first strip is drawn along the longitudinal direction such that the first end portion moves into the first cavity, the first end portion is squeezed by the squeezing mechanism to squeeze out the fluid substrate received thereat to be transferred to the volumetric pool, for example via the transfer canal. That is, as discussed, the first strip may be moved along the longitudinal direction. Once, the fluid substrate is received on the first end portion, the second end portion of the first strip may be pulled so that the first end portion moves into the first cavity. As the first end portion is moved between the squeezing mechanism, the fluid substrate received at the first end portion may get squeezed out and may be transferred to the volumetric pool, for example, via the transfer canal. The volumetric pool may store only the desired volume of the fluid substrate which is equivalent to the defined limited internal volume thereof. The fluid substrate excess to the limited internal volume of the volumetric pool overflows from the volumetric pool, in order to retain only the desired volume of the fluid substrate in the volumetric pool equivalent to the limited internal volume. That is, as discussed, the volumetric pool may store only exact volume of the fluid substrate equivalent to its limited internal volume. If the fluid substrate is excess to the limited internal volume of the volumetric pool, the excess fluid substrate may overflow from the volumetric pool, for example via the overflow channels to the excess pool, so as to maintain the desired volume of the fluid substrate in the volumetric pool.

Optionally, the apparatus further comprises a second cavity formed in the housing, the second cavity extending along a portion of the first length in the longitudinal direction therein. Herein, the second cavity may be in the form of a protrusion formed in the housing along the portion of the first length in the longitudinal direction. In the housing, the second cavity may be parallel to the first cavity and may be offset from the first cavity in the lateral direction at some distance.

Optionally, the apparatus further comprises a second strip having a third end portion and a fourth end portion, the second strip supported in the second cavity such that the third end portion thereof is arranged at a third height above the second height of the volumetric pool. The second strip may be an assay strip assembled in production. Typically, the assay strip may be of 60 millimetres length with standard lateral flow strip components and may be manufactured with standard strip manufacturing capabilities as known in the art. The second strip may have the third end portion and the fourth end portion. The second strip may be arranged on the second cavity such that, the third end portion is elevated at the third height above the second height of the volumetric pool. For this purpose, the housing may provide one or more supporting elements to keep the third end portion of the second strip stays at the elevated position at the third height. In the apparatus, the volumetric pool may be located below the third end portion of the second strip in the housing.

The apparatus further comprises a latch button provided with the housing above a location of the third end portion of the second strip when supported in the second cavity, the latch button adapted to be pressed opposite to the vertical direction to cause the third end portion to bend from the third height to below the second height and thereby be disposed in contact with the desired volume of the fluid substrate in the volumetric pool. The latch button may be in the form of a push button which may flex when pushed opposite to the vertical direction. In the present apparatus, the latch button is used to activate the diagnostic testing process provided thereby. The latch button may be positioned above the location of the third end portion of the second strip, so that when the latch button is pushed, at least about fifty percent to its possible flex movement, the third end portion of the second strip may bend from the third height to the second height (or even below thereof) and may thus come in contact with the desired volume of the fluid substrate in the volumetric pool. The third end portion of the second strip may then absorb the desired volume of the fluid substrate from the volumetric pool and the testing process may thus be activated. In some examples, a latching mechanism associated with the latch button may help to keep the latch button, and thereby the second strip in bent orientation for proper absorption of the fluid substrate from the volumetric pool.

Optionally, the second cavity, the latch button and at least a portion of the volumetric pool are offset from the first cavity in the lateral direction. That is, the second cavity, the latch button and at least a portion of the volumetric pool may be disposed at some distance to each other from the first cavity in the lateral direction. In an embodiment, at least some portion of the volumetric pool may be located below the third end portion of the second strip. In such a case, only the at least portion of the volumetric pool may be offset from the first cavity in the lateral direction. In an alternative embodiment, the entire volumetric pool may be located below the third end portion of the second strip without any limitations. In such a case, the entire volumetric pool may be offset from the first cavity in the lateral direction. As discussed, the latch button may be disposed above the location of the third end portion of the second strip. Since, the second cavity is offset from the first cavity in lateral direction, it may be contemplated that the latch button may also be offset from the first cavity in the lateral direction.

Optionally, the apparatus further comprises a buffer pool located adjacent to the volumetric pool in the housing and storing a solution therein, wherein the latch button is adapted to be further pressed to cause the third end portion to bend further to be disposed in contact with the solution in the buffer pool. The buffer pool may be in the form of a pouch, a cavity or a blister that may be located adjacent to the volumetric pool and may store the solution (buffer solution). Herein, the solution may be water, colour-forming indicator solution such as, substrate for enzymatic reaction and the like. In the present apparatus, the latch button may be pressed further to 100 percent of its flex movement in order to further bend the third end portion of the second strip and thereby be disposed to be in contact with the solution in the buffer pool. Optionally, the buffer pool may be the pouch that may be punctured via the latch button when pressed fully. In some embodiments, a second activation button may be provided to puncture the buffer pool.

Optionally, the apparatus further comprises an electrical circuit operatively connected to the latch button, a timer and one or more of a light emitting diode and a speaker, wherein the electrical circuit is configured to: activate at least the timer upon pressing of the latch button, with the timer configured to generate a time signal after completion of a predefined time-period from activation thereof; and activate at least one of the light emitting diode and the speaker in response to generation of the time signal. As discussed, the electrical circuit may be connected to the latch button, the timer and one or more of the light emitting diode and the speaker. The electrical circuit may also include single-use battery such as paper-based batteries for supplying power. As the latch button is pressed, say by about fifty percent, the testing process may be activated and hence, the timer may be activated. Herein, the latch button may have a mechanical arrangement to activate the electrical circuit. Optionally, pulling of the first strip may be used to activate the electrical circuit for monitoring purposes. It may be appreciated that once the testing process is activated, the testing result may be read after a time duration of about 1 to 30 minutes that may be necessary for the testing process to complete. Hence, the timer may be set to the time duration and may start counting down from the time duration when the testing process is activated. Tactile, audible and visible indication of assay incubation starting time may also be provided. For example, the time duration of ten minutes may be set on the timer to indicate when the test result is ready to monitor. Once, the time duration lapses, the timer may send the time signal to activate at least one of the: light emitting diode and the speaker in response to generation of the time signal. In an embodiment, the light emitting diode may glow to indicate that the time duration has lapsed and the test result may be read. In another embodiment, the speaker may generate a sound such as, a bell to indicate that the time duration has lapsed and the test result may be read. In some embodiments, both the light emitting diode and the speaker may be activated in response to the generation of the time signal. Optionally, the apparatus may allow multiple samples of the fluid substrates in separate monitoring apparatus collected at different time points to be activated at the same time, using the respective electrical circuits.

Optionally, the first strip comprises a first status indicator printed at the first end portion thereof and optionally a second status indicator printed at the second end portion thereof. Herein, the first status indicator may indicate that the first strip is "UNUSED" and the second status indicator may indicate that the first strip is "USED". In an embodiment, the first strip may comprise the first status indicator printed at the first end portion or proximal thereto that may indicate that the first strip is UNUSED. Once, the first strip is used for testing purposes, the first end portion may be moved inside the housing and the first status indicator may be hidden indicating that the first strip is used. In an alternative embodiment, the second status indicator that may indicate that the strip is USED may be printed at the second end portion of the first strip. When the first strip is unused, the second status indicator may be hidden inside the housing indicating that the first strip is UNUSED. When the first strip is used by pulling the second end portion, the second status indicator may be visible outside the housing indicating that the strip is USED. In some embodiments, the first status indicator and the second status indicator may be coloured indicators either one of which may be visible before and after the first strip is pulled through the first cavity indicating that the strip is UNUSED and USED, respectively.

Optionally, the housing is a unitary structure. The unitary structure of the housing may make the housing compact so that the first strip, the second strip, the buffer pool, the volumetric pool, the electrical circuit, the timer, the LED and the speaker are all assembled in the same unit that is the housing. Moreover, the latch button may also be formed in the unitary structure. This makes the apparatus compact and easy to use. Such housing may be formed by using 3D printing technique, casting technique or the like.

The apparatus may also include a read-out window that may be formed in the housing, positioned corresponding to a fourth end portion of the second strip. The read-out window may be in the form of a monitoring window from which the test result may be read after the time duration has lapsed. The test result may generate indicative test and control line signals with visual, fluorescent or other signal generator technology on the read-out window. That is, the test result may be indicated on the readout window where lines appearing on the second strip may be read. For example, after ten minutes of activating the testing process has lapsed, the test result in the form of lines or other visual elements, such as, colour, fluorescence signal and the like may appear on the second strip that may be read via the read-out window. The test result may include a control line used as a zero value for positioning and a test line.

For example, in an embodiment, the test result may include the control line at zero position and the test line at a relative location of six millimetres from the control line. Location of the subtracted background signal may be 7 to 8 millimetres. The test result may be read through the read-out window by a manual check, specific adapter or common adapters, fluorometric reader instruments, magnetic field-based reader instruments, amperometry or electronic conductivity-based reader instruments. Optionally, a smartphone app may be used to monitor test result. Herein, a photograph may be taken by the app on the smartphone. The photo may be sent to a server for analysis and the server may return analysed testing results to the app in the smartphone.

The apparatus may be used for any health and wellbeing monitoring applications where measurements samples in the form of the fluid substrate are based, for example, on a tiny sample of saliva for monitoring cortisol level and identifying stress triggers of humans. In practice first, the first end portion of first strip may be inserted in the saliva collection tube or directly into the mouth. Second, the second end portion of the first strip may be pulled through the housing to introduce the received fluid substrate into the volumetric pool in the housing. Third, the latch button may be pressed to bend the second strip to be disposed in contact with the fluid substrate in the volumetric pool. Herein, the latching mechanism may keep the second strip in bent orientation. Fourth, after a pre-set time of typically 10 minutes, the testing results may be read on the readout window or the like.

Moreover, the present description also relates to the method for diagnostic testing. The method is implemented by using an immunochromatographic diagnostic test kit. Various embodiments and variants disclosed above apply *mutatis mutandis* to the present method as described hereinafter.

The method comprises providing a housing having a first cavity to support a first strip with a first end portion thereof located outside the housing and a second cavity to support a second strip with a third end portion thereof located inside the housing, receiving a fluid substrate at the first end portion of the first strip when supported in the first cavity and drawing the first strip along the longitudinal direction such that the first end portion moves into the first cavity to be squeezed by a squeezing mechanism arranged in the housing, to squeeze out the fluid substrate received thereat to be transferred to a volumetric pool formed in the housing via a transfer canal. Herein, the volumetric pool has a limited internal volume to cause the fluid substrate excess to the limited internal volume of the volumetric pool to overflow from the volumetric pool, to retain a desired volume of the fluid substrate in the volumetric pool equivalent to the limited internal volume.

Optionally, the method further comprises pressing a latch button provided with the housing above a location of the third end portion of the second strip when supported in the second cavity, to cause the third end portion to bend and be disposed in contact with the desired volume of the fluid substrate in the volumetric pool.

Optionally, the method further comprises supporting the housing between lips of a user and impelling the first strip supported in the first cavity into a mouth of the user, to receive the fluid substrate therefrom.

The apparatus and the method of the present disclosure provides self-contained sample collection functions with sample volume quantification. The apparatus and the method of the present disclosure ensure that an exact volume of a fluid substrate is collected and then be used for diagnostic testing purposes. In the present apparatus, the volumetric pool, the first strip and the second strip are all contained within the housing making the apparatus compact. Moreover, the apparatus is easy to manufacture and may be manufactured with existing machinery and material components. Furthermore, the apparatus is a single package system with reduced packing materials and cleanly disposable wastes as opposed to conventional ones where several different separate components have been used. The apparatus and the method may be used for number of applications where the fluid substrate such as, saliva, blood, urine, food, drink, water, brewing process sample, industrial process sample and the like may be used for indicative test and control line signals with visual, fluorescent or other signal generator technologies may appear.

### DETAILED DESCRIPTION OF DRAWINGS

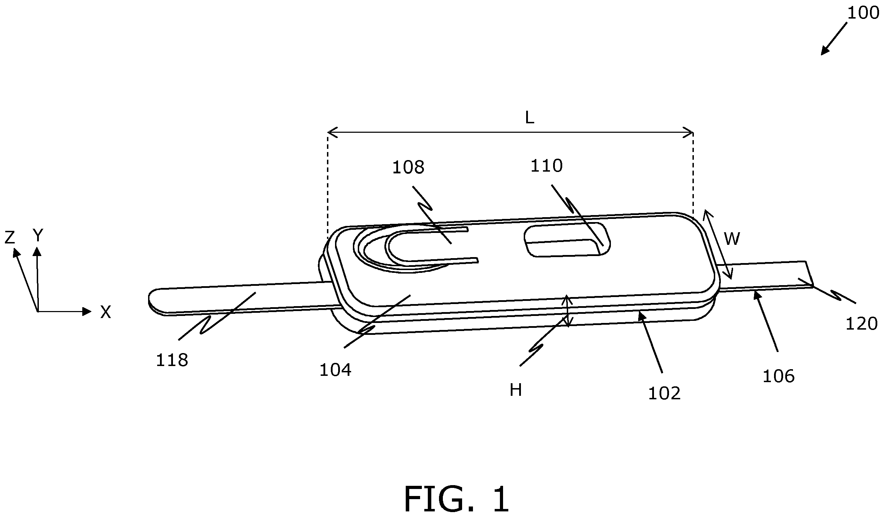

FIG. 1 is a perspective view illustration of an apparatus **100,** in accordance with an embodiment of the present disclosure. As illustrated, the apparatus **100** includes a housing **102** supporting a first strip **106,** and providing a latch button **108** and a read-out window **110.** The housing **102** has a first length **L** along a longitudinal direction **X,** a first height **H** along a vertical direction **Y** and a first width **W** along a lateral direction **Z.** The first strip **106** has a first end portion **118** and a second end portion **120.** The first end portion **118** is located outside of the housing **102** and is adapted to receive a fluid substrate.

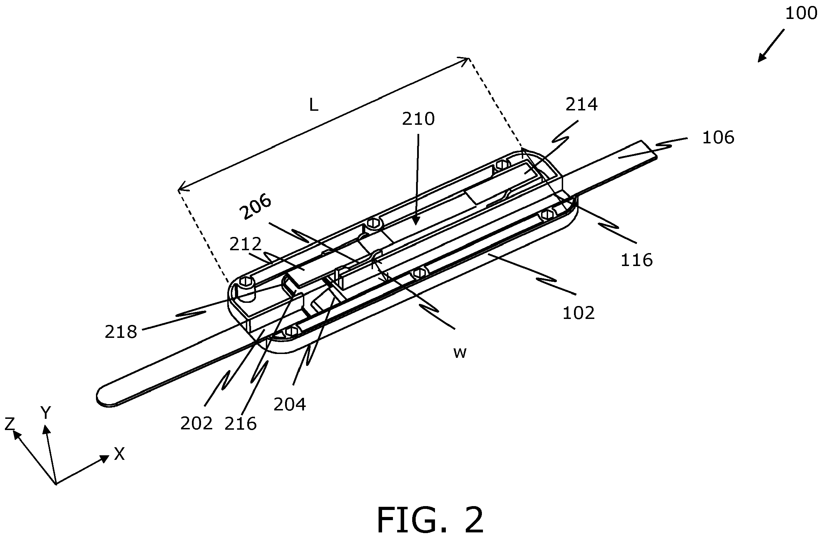

FIG. 2 is a perspective view illustration of the apparatus illustrated in FIG. 1 with a top section removed therefrom, in accordance with an embodiment of the present disclosure. It may be observed from FIG. 2 that the housing **102** includes a first cavity **202,** a squeezing mechanism **204** and a second cavity **206.** The first cavity **202** extends along the first length **L** in the longitudinal direction **X** and has a second width **w** along the lateral direction **Z.** The first cavity **202** is adapted to support the first strip **106** to be movable along the longitudinal direction **X.** The squeezing mechanism **204** is disposed corresponding to at least a portion of the second width **w** of the first cavity **202** in the lateral direction **Z.** The second cavity **206** extends along a portion of the first length **L** in the longitudinal direction **X.** A second strip **210** having a third end portion **212** and a fourth end portion **214** is supported in the second cavity **206.** Further, as illustrated, the housing **102** includes a volumetric pool **216.** The volumetric pool **216** has a sidewall **218** extending to a second height (not labelled) along the vertical direction **Y** to define a limited internal volume for the volumetric pool **216.**

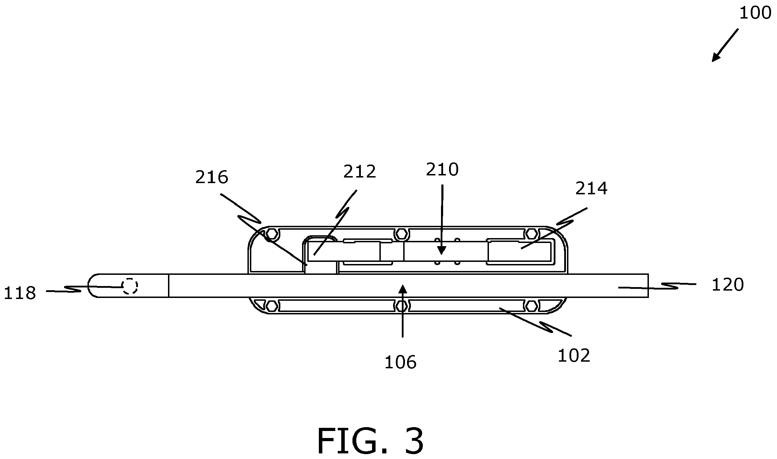

FIG. 3 is a top planar view illustration of the apparatus illustrated in FIG. 1 with a top section removed therefrom, in accordance with an embodiment of the present disclosure. As illustrated, the second strip **210** is supported in the second cavity (such as, the second cavity **206** of FIG. 2) such that the third end portion **212** is arranged at a third height (not labelled) above the second height of the volumetric pool **216.**

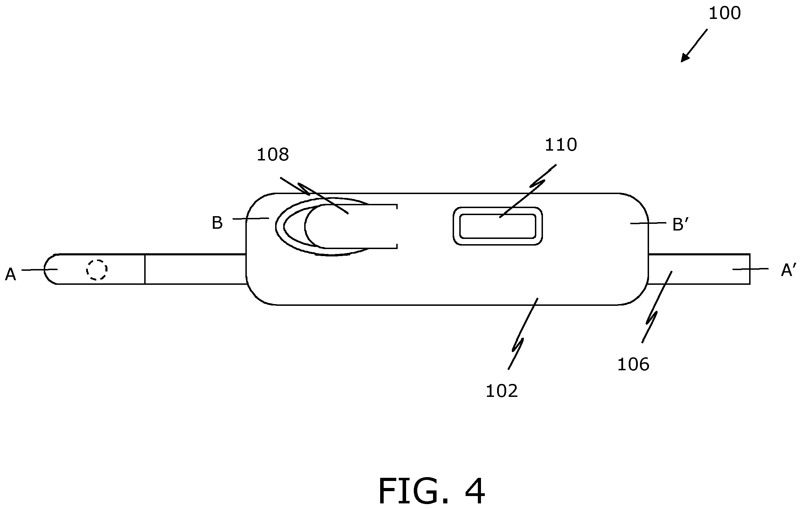

FIG. 4 is a top planar view illustration of the apparatus **100** of FIG. 1, in accordance with an embodiment of the present disclosure. As illustrated, the apparatus **100** includes the housing **102,** the first strip **106,** the latch button **108** and the read-out window **110,** with planes A-A' and B-B' marked on the apparatus **100** through which the apparatus **100** may be sectioned, as described below.

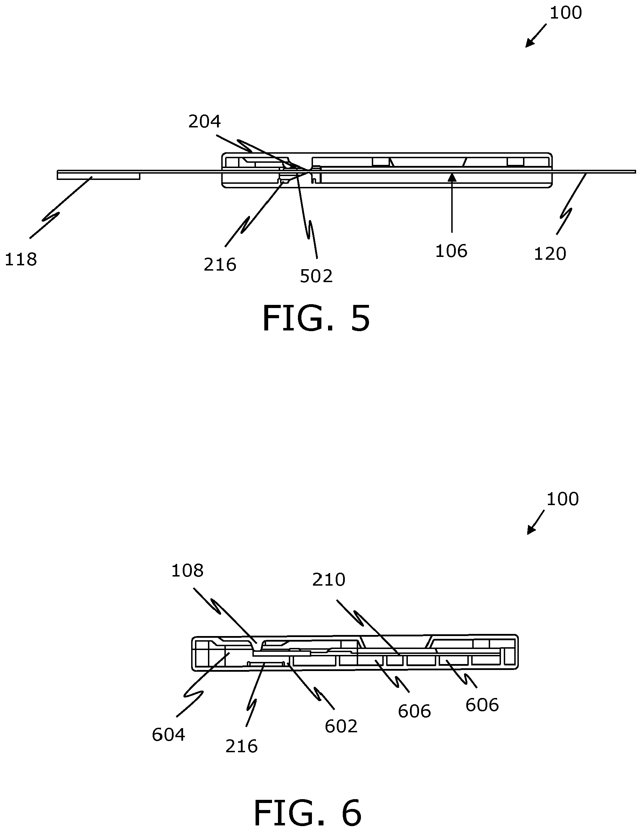

FIG. 5 is a section view illustration of the apparatus **100** sectioned along the plane A-A' of FIG. 4, in accordance with an embodiment of the present disclosure. It may be observed from FIG. 5 that the first strip **106** has varying thickness. The first end portion **118** of the first strip **106** is thicker than the second end portion **120** of the first strip **106.** Hence, when the second end portion **120** of the first strip **106** is pulled such that the first end portion **118** moves between the squeezing mechanism **204,** the first end portion **118** gets squeezed in constriction of space provided by the squeezing mechanism **204.** As illustrated, a transfer canal **502** is disposed in a region in the first cavity (similar to the first cavity **202** of FIG. 2) proximal to the squeezing mechanism **204** in fluid communication with the volumetric pool **216** in order to transfer the squeezed fluid substrate to the volumetric pool **216.**

FIG. 6 is a section view illustration of the apparatus **100** sectioned along the plane B-B' of FIG. 4, in accordance with an embodiment of the present disclosure. As illustrated, the apparatus **100** includes an excess pool **602** which is in fluid communication with the volumetric pool **216** via one or more overflow channels **604.** The one or more overflow channels **604** are disposed in fluid communication with the sidewall (such as, the sidewall **218** of FIG. 2) of the volumetric pool **216** at its second height. The fluid substrate excess to the limited internal volume of the volumetric pool **216** overflows from the volumetric pool **216** to the excess pool **602** via the one or more overflow channels **604.** As discussed, volumetric pool **216** has the limited internal volume. The fluid substrate excess to the limited internal volume of the volumetric pool overflows from the volumetric pool **216** and get stored in the excess pool **602.** The apparatus **100** includes one or more supporting elements **606** to keep the second strip **210** in the elevated position. When the latch button **108** is pressed opposite to the vertical direction by about fifty percent, third end portion (such as, the third end portion **212** of FIG. 2) of the second strip **210** bends from the third height to below the second height and is disposed in contact with desired volume of the fluid substrate in the volumetric pool **216.**

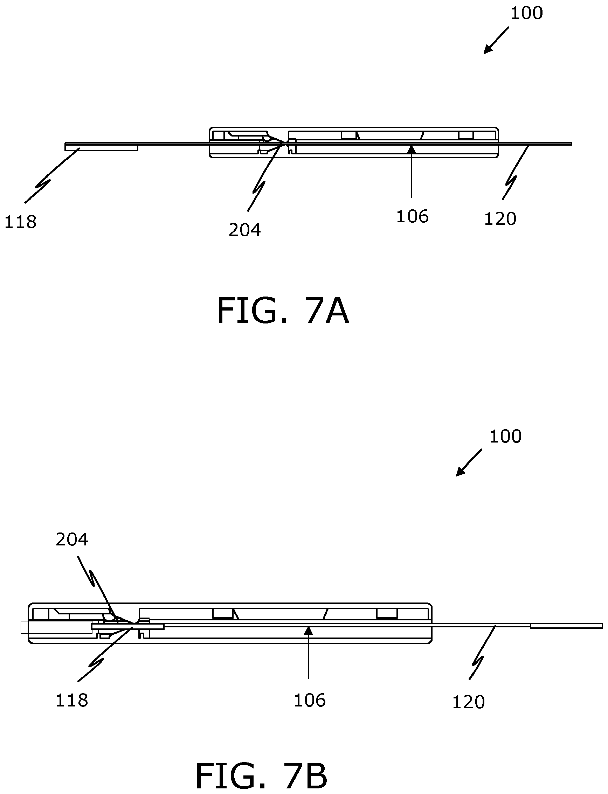

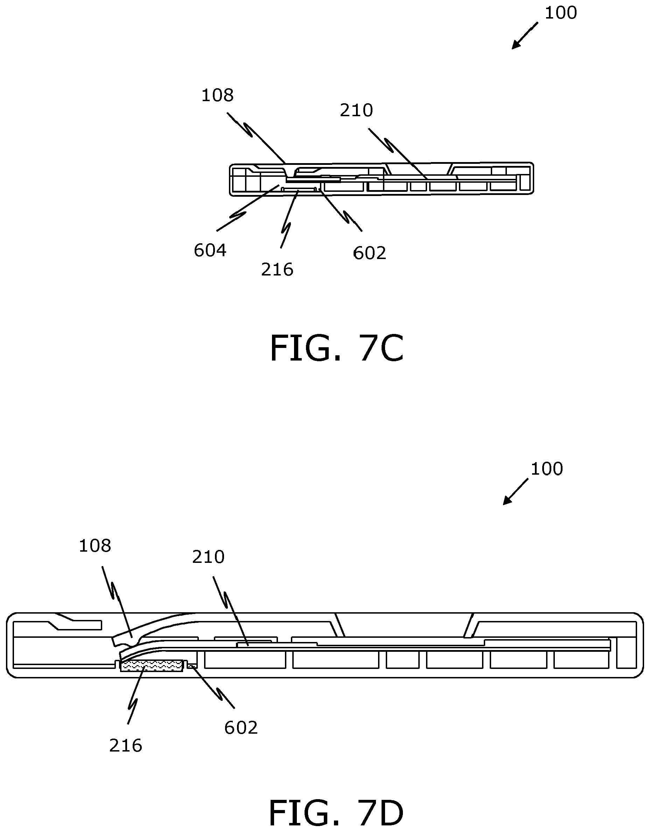

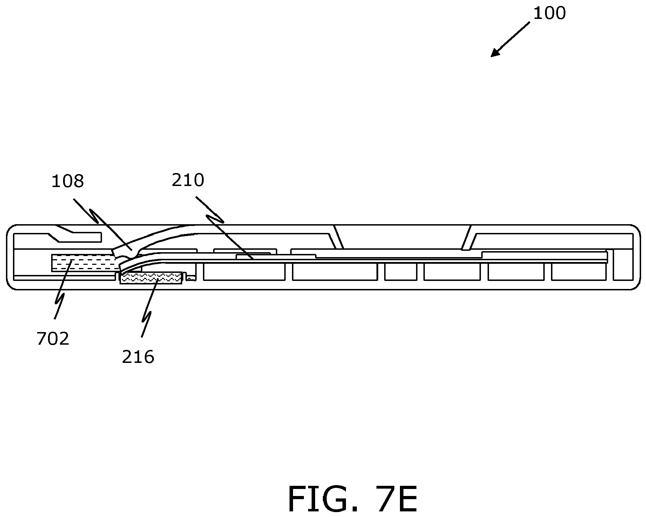

FIGs. 7A-7E are section view illustration of the apparatus **100** for illustrating step by step process of operating the apparatus **100,** in accordance with an embodiment of the present disclosure. Referring to FIG. 7A, the first end portion **118** and the second end portion **120** of the first strip **106** are outside the housing (such as, the housing **102** of FIG. 1). The fluid substrate may be received on the first end portion **118.** Referring to FIG. 7B, the second end portion **120** of the first strip **106** is pulled so that the first end portion **118** moves inside the housing (such as, the housing **102** of FIG. 1) between the squeezing mechanism **204** which provides constriction in space for squeezing the first end portion **118.** Referring to FIG. 7C, due to squeezing of the first end portion (such as, first end portion **118** of FIG. 1), the fluid substrate received at first end portion (such as, first end portion **118** of FIG. 1) is squeezed and transferred to the volumetric pool **216.** Excess fluid substrate is collected in the excess pool **602** via one or more overflow channels (such as, the overflow channels **604** of FIG. 6). Referring to FIG. 7D, the latch button **108** is pressed by about 50 percent opposite to the vertical direction which pushes third end portion (such as, the third end portion **212** of FIG. 2) of the second strip **210** so that the third end portion (such as, the third end portion **212** of FIG. 2) bends from the third height to below the second height and thereby is disposed in contact with desired volume of the fluid substrate in the volumetric pool **216.** Herein, the diagnostic testing process provided by the apparatus **100** is activated. Referring to FIG. 7E, the apparatus **100** includes a buffer pool **702.** The buffer pool **702** is located adjacent to the volumetric pool **216** in the housing (such as, the housing **102** of FIG. 1) and stores a solution therein. The latch button **108** is adapted to be further pressed by about 100 percent to cause the third end portion (such as, the third end portion **212** of FIG. 2) of the second strip **210** to bend further to be disposed in contact with the solution in the buffer pool **702.**

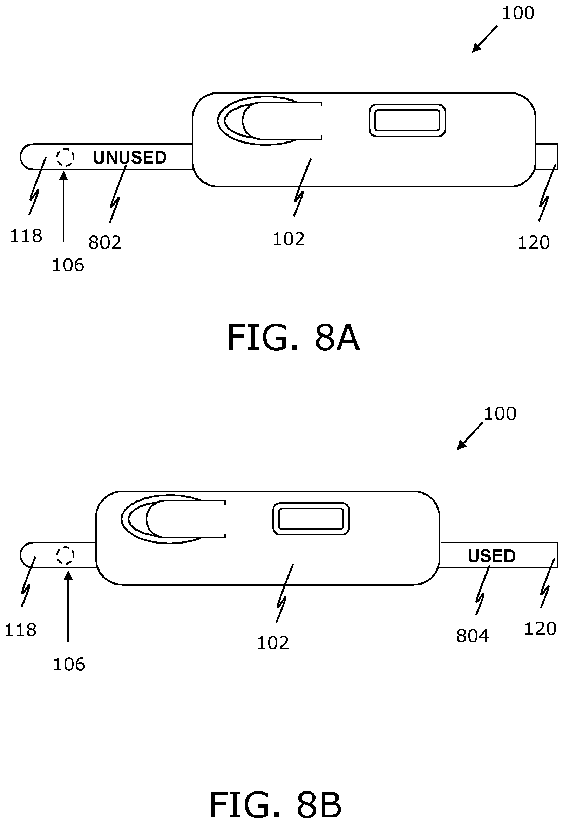

FIG. 8A is a top planar view illustration of the apparatus **100** with the first strip **106** in a position depicting first status indicator **802,** in accordance with an embodiment of the present disclosure. The first status indicator **802** is printed at the first end portion **118** of the first strip **106.** In the illustrated example, the first status indicator **802** is "UNUSED" and indicates that the first strip **106** is not used. It may be observed that when the first strip **106** is pulled from the second end portion **120,** the first status indicator **802** may be moved inside the housing **102** and hence, may be hidden. FIG. 8B is a top planar view illustration of the apparatus **100** with the first strip **106** in a position depicting a second status indicator **804,** in accordance with an embodiment of the present disclosure. The second status indicator **804** is printed at the second end portion **120** of the first strip **106.** In the illustrated example, the second status indicator **804** is "USED" and indicates that the first strip **106** has been used. The second status indicator **804** may be visible only when the first strip **106** has been used by pulling though the second end portion **120.**

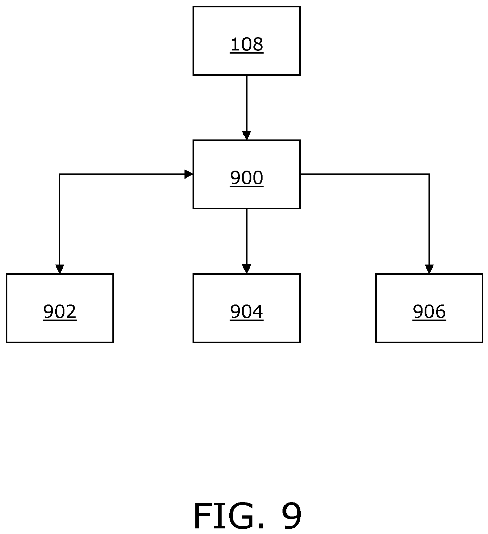

FIG. 9 is a schematic illustration of an electrical circuit **900,** in accordance with an embodiment of the present disclosure. As illustrated, the electrical circuit **900** is operatively connected to the latch button **108,** a timer **902** and one or more of a light emitting diode **904** and a speaker **906.** The electrical circuit **900** is configured to activate at least the timer **902** upon pressing of the latch button **108,** with the timer **902** configured to generate a time signal after completion of a predefined time-period from activation thereof. The electrical circuit **900** is further configured to activate at least one of: the light emitting diode **904** and the speaker **906** in response to generation of the time signal.

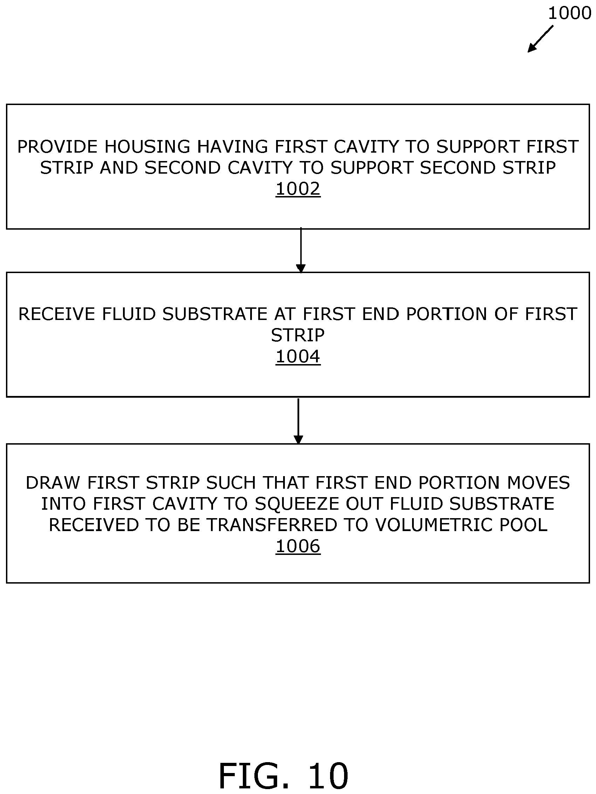

FIG. 10 is a flowchart listing steps involved in method **1000** for diagnostic testing, in accordance with an embodiment of the present disclosure. The method **1000** includes, at step **1002,** providing a housing having a first cavity to support first strip and a second cavity to support a second strip. Herein, the first cavity supports the first strip with a first end portion thereof located outside the housing and the second cavity supports the second strip with a third end portion thereof located inside the housing. The method **1000** includes, at step **1004,** receiving a fluid substrate at the first end portion of the first strip when supported in the first cavity. The method **1000** includes, at step **1006,** drawing the first strip such that the first end portion moves into the first cavity to squeeze out the fluid substrate received to be transferred to volumetric pool. The first strip is drawn along the longitudinal direction such that the first end portion moves into the first cavity to be squeezed by a squeezing mechanism arranged in the housing in order to squeeze out the fluid substrate received thereat to be transferred to a volumetric pool formed in the housing via a transfer canal. Herein, the volumetric pool has a limited internal volume to cause the fluid substrate excess to the limited internal volume of the volumetric pool to overflow from the volumetric pool, to retain a desired volume of the fluid substrate in the volumetric pool equivalent to the limited internal volume.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Expressions such as "may" and "can" are used to indicate optional features, unless indicated otherwise in the foregoing. Reference to the singular is also to be construed to relate to the plural.

## Claims

1. An apparatus (100) for diagnostic testing, the apparatus comprising:
- a housing (102) having a first length (L) along a longitudinal direction (X) thereof, a first height (H) along a vertical direction (Y) thereof and a first width (W) along a lateral direction (Z) thereof;
- a first cavity (202) formed in the housing, the first cavity extending along the first length in the longitudinal direction and having a second width (w) along the lateral direction, the first cavity adapted to support a first strip (106) to be movable along the longitudinal direction therein, the first strip having a first end portion (118) and a second end portion (120) with the first end portion thereof located outside of the housing and adapted to receive a fluid substrate thereat when supported in the first cavity;
- a volumetric pool (216) formed in the housing, the volumetric pool having a sidewall (218) extending to a second height along the vertical direction to define a limited internal volume for the volumetric pool;
- a squeezing mechanism (204) arranged in the housing, the squeezing mechanism disposed corresponding to at least a portion of the second width of the first cavity in the lateral direction; and
- wherein when the first strip is drawn along the longitudinal direction such that the first end portion moves into the first cavity, the first end portion is squeezed by the squeezing mechanism to squeeze out the fluid substrate received thereat to be transferred to the volumetric pool, and
- wherein the fluid substrate excess to the limited internal volume of the volumetric pool overflows from the volumetric pool, to retain a desired volume of the fluid substrate in the volumetric pool equivalent to the limited internal volume.

2. An apparatus (100) according to claim 1 further comprising one or more overflow channels (604) formed in the housing (102), the one or more overflow channels disposed in fluid communication with the sidewall (218) of the volumetric pool (216) at the second height thereof and with an excess pool (602) formed in the housing, wherein the fluid substrate excess to the limited internal volume of the volumetric pool overflows from the volumetric pool to the excess pool via the one or more overflow channels.

3. An apparatus (100) according to any one of claims 1 or 2 further comprising a second cavity (206) formed in the housing (102), the second cavity extending along a portion of the first length (L) in the longitudinal direction (X) therein.

4. An apparatus (100) according to claim 3 further comprising a second strip (210) having a third end portion (212) and a fourth end portion (214), the second strip supported in the second cavity (206) such that the third end portion thereof is arranged at a third height above the second height of the volumetric pool (216).

5. An apparatus (100) according to claims 3-4 further comprising a latch button (108) provided with the housing (102) above a location of the third end portion (212) of the second strip (210) when supported in the second cavity (206, the latch button adapted to be pressed opposite to the vertical direction (Y) to cause the third end portion to bend from the third height to below the second height and thereby be disposed in contact with the desired volume of the fluid substrate in the volumetric pool (216).

6. An apparatus (100) according to claim 5, wherein the second cavity (206), the latch button (108) and at least a portion of the volumetric pool (216) are offset from the first cavity (202) in the lateral direction (Z).

7. An apparatus (100) according to any one of claims 5 or 6 further comprising a buffer pool (702) located adjacent to the volumetric pool (216) in the housing (102) and storing a solution therein, wherein the latch button (108) is adapted to be further pressed to cause the third end portion (212) to bend further to be disposed in contact with be disposed in contact with the solution in the buffer pool.

8. An apparatus (100) according to any one of claims 5-7 further comprising an electrical circuit (900) operatively connected to the latch button (108), a timer (902) and one or more of a light emitting diode (904) and a speaker (906), wherein the electrical circuit is configured to:
activate at least the timer upon pressing of the latch button, with the timer configured to generate a time signal after completion of a predefined time-period from activation thereof; and
activate at least one of the light emitting diode and the speaker in response to generation of the time signal.

9. An apparatus (100) according to any one of preceding claims, wherein the first strip (106) comprises a first status indicator (802) printed at the first end portion (118) thereof and optionally a second status indicator (804) printed at the second end portion (120) thereof.

10. An apparatus (100) according to any one of preceding claims, wherein the housing (102) is a unitary structure.

11. An apparatus (100) according to any one of preceding claims, wherein the apparatus is an immunochromatographic diagnostic test kit.

12. An apparatus (100) according to any one of preceding claims, wherein the defined limited internal volume of the volumetric pool (216) is in a range of 10 µL to 200 µL.

13. A method (1000) for diagnostic testing, the method comprising:
- providing a housing (102) having a first cavity (202) to support a first strip (106) with a first end portion (118) thereof located outside the housing and a second cavity (206) to support a second strip (210) with a third end portion (212) thereof located inside the housing;
- receiving a fluid substrate at the first end portion of the first strip when supported in the first cavity; and
- drawing the first strip along the longitudinal direction (X) such that the first end portion moves into the first cavity to be squeezed by a squeezing mechanism (204) arranged in the housing, to squeeze out the fluid substrate received thereat to be transferred to a volumetric pool (216) formed in the housing,
- wherein the volumetric pool has a limited internal volume to cause the fluid substrate excess to the limited internal volume of the volumetric pool to overflow from the volumetric pool, to retain a desired volume of the fluid substrate in the volumetric pool equivalent to the limited internal volume.

14. A method (1000) according to claim 13 further comprising pressing a latch button (108) provided with the housing (102) above a location of the third end portion (212) of the second strip (210) when supported in the second cavity (206), to cause the third end portion to bend and be disposed in contact with the desired volume of the fluid substrate in the volumetric pool (216).

15. A method (1000) according to any one of claims 13 or 14 further comprising:
- supporting the housing (102) between lips of a user; and
- impelling the first strip (106) supported in the first cavity (202) into a mouth of the user, to receive the fluid substrate therefrom.
